(19) Europäisches Patentamt
European Patent Office
Office européen des brevets

(11) **EP 1 011 858 B2**

(12) **NOUVEAU FASCICULE DE BREVET EUROPEEN**
Après la procédure d'opposition

(45) Date de publication et mention de la décision concernant l'opposition:
**16.12.2020 Bulletin 2020/51**

(45) Mention de la délivrance du brevet:
**16.03.2011 Bulletin 2011/11**

(21) Numéro de dépôt: **98942751.3**

(22) Date de dépôt: **18.08.1998**

(51) Int Cl.:
***B01J 20/18*** *(2006.01)*          ***C07C 15/08*** *(2006.01)*
***C07C 7/13*** *(2006.01)*

(86) Numéro de dépôt international:
**PCT/FR1998/001818**

(87) Numéro de publication internationale:
**WO 1999/010096 (04.03.1999 Gazette 1999/09)**

(54) **ADSORBANTS ZEOLITIQUES AGGLOMERES LEUR PROCEDE D'OBTENTION ET LEUR UTILISATION POUR L'ADSORPTION DE PARAXYLENE A PARTIR DE COUPES DE C8 AROMATIQUES**

AGGLOMERIERTE ZEOLITISCHE ADSORPTIONSMITTEL, VERFAHREN ZU IHRER HERSTELLUNG UND IHRE VERWENDUNG BEI DER ADSORPTION VON PARA-XYLOL AUS C8 AROMATISCHEN KOHLENWASSERSTOFFSCHNITTEN

AGGLOMERATED ZEOLITE ADSORBENTS, METHOD FOR OBTAINING THEM AND USE FOR ADSORBING PARAXYLENE FROM AROMATIC C8 CUT FRACTIONS

(84) Etats contractants désignés:
**DE ES FR GB IT PT**

(30) Priorité: **21.08.1997 FR 9710535**

(43) Date de publication de la demande:
**28.06.2000 Bulletin 2000/26**

(60) Demande divisionnaire:
**07102072.1**

(73) Titulaires:
• **ARKEMA FRANCE**
  **92700 Colombes (FR)**
• **IFP Energies nouvelles**
  **92500 Rueil-Malmaison (FR)**

(72) Inventeurs:
• **PLEE, Dominique**
  **F-64140 Lons (FR)**
• **METHIVIER, Alain**
  **F-92500 Rueil-Malmaison (FR)**

(74) Mandataire: **Bandpay & Greuter**
  **30, rue Notre-Dame des Victoires**
  **75002 Paris (FR)**

(56) Documents cités:
**EP-A- 0 531 191        EP-B1- 0 196 043**
**WO-A1-99/10096      US-A- 2 882 243**
**US-A- 2 882 244      US-A- 2 882 244**
**US-A- 2 985 589      US-A- 3 130 007**
**US-A- 3 558 730      US-A- 3 558 732**
**US-A- 3 663 638      US-A- 3 734 974**
**US-A- 3 878 127**

• **C. G. Coe; S. M. Kuznicki; R. Srinivasan; R. J. Jenkins: "Molecularly Engineered, High-Performance Adsorbent", ACS Symposium Series, American Chemical Society/Oxford University Press, US, 1 January 1988 (1988-01-01), pages 478-491, US ISSN: 0097-6156**
• **Dyer, A.: "An Introduction to zeolite molecular sieves", 1988 pages 38-51,**
• **Breck, Donald: "Zeolite Molecular Sieves -Structure, chemistry and use", 1974 pages 344,353,368-369,529-565,**

**EP 1 011 858 B2**

**Description**

DOMAINE TECHNIQUE

Le domaine de l'invention est celui des adsorbants zéolitiques pour la séparation des xylènes, en particulier en vue de la production industrielle de paraxylène.

TECHNIQUE ANTERIEURE

L'industrie réclame du paraxylène de haute pureté, entre autres pour sa transformation en acide téréphtalique destiné à la fabrication du PET.
L'art antérieur a reconnu que les adsorbants constitués de zéolites X ou Y échangées au moyen d'ions tels que le baryum, le potassium ou le strontium, seuls ou en mélange, sont efficaces pour adsorber sélectivement le paraxylène dans un mélange contenant au moins un autre isomère aromatique en $C_8$. Les brevets US 3.558.730, US 3.558.732, US 3.626.020 et US 3.663.638 divulguent des adsorbants comprenant des aluminosilicates échangés par du baryum et du potassium qui séparent efficacement le paraxylène d'un mélange d'isomères aromatiques en $C_8$. Ces adsorbants sont utilisés comme agents d'adsorption dans les procédés en phase liquide, de préférence de type contre-courant simulé similaires à ceux décrits dans US 2,985,589, qui s'appliquent entre autres aux coupes de $C_8$ aromatiques issues, par exemple, des procédés de dialkylation du benzène, dans les procédés en phase gazeuse.
Les performances du procédé industriel de séparation du paraxylène dépendent pour une bonne part de l'adsorbant, de sa capacité d'adsorption et de la sélectivité qu'il montre pour le paraxylène dans un milieu constitué d'aromatiques en $C_8$, typiquement de paraxylène lui-même (PX), de métaxylène (MX), d'orthoxylène (OX), d'éthylbenzène (EB), ainsi que de l'aptitude des désorbants, tels le toluène et le paradiéthylbenzène, à en désorber le paraxylène adsorbé.
On définit la sélectivité Sél(B/A) de l'adsorbant pour un composé (B) par rapport à un composé (A) comme le rapport des concentrations des composés dans la phase adsorbée divisé par le rapport des concentrations des composés dans la phase non adsorbée à l'équilibre.
L'équation de la sélectivité est la suivante :

$$Sél(B/A) = \frac{(B)z / (A)z}{(B)s / (A)s}$$

où (B)z et (B)s représentent les concentrations de B respectivement dans la zéolite et dans la solution,
où (A)z et (A)s représentent les concentrations de A dans la zéolite et la solution. La méthode d'appréciation de ces grandeurs est exposée plus loin.
Les zéolites qu'on rencontre dans l'art antérieur pour la séparation des xylènes appartiennent au type structural de la faujasite, tout d'abord décrites dans US 2.882.244 et US 3.130.007, qui sont des silico-aluminates cristallisés possédant des cages de taille parfaitement déterminée connectées dans les trois dimensions. Les faujasites répondent à la formule générale :

(1 $\pm$ 0,1) $M_{2/n}O$ : $Al_2O_3$ ; $W\ SiO_2$ ; $Y\ H_2O$

dans laquelle

M représente au moins un cation alcalin ou alcalino-terreux de valence n,
Y est inférieur ou égal à 8 selon la nature de M et le degré d'hydratation du cristal,
W est le facteur qui permet de faire une distinction entre les faujasites riches en silice (faujasites Y) et les faujasites riches en alumine (faujasites X). On convient de situer les faujasites X dans le domaine W $\leq$ 3, et les faujasites Y dans le domaine W > 3, ce qu'on interprète plutôt selon le rapport atomique Si/Al de part et d'autre de 1,5. Au sens de la présente invention, on introduit une distinction supplémentaire commode avec les faujasites à faible teneur en silice (qu'on dira LSX, abréviation dans laquelle l'homme du métier lit Low Silica X), pour W < 2,3 (Si/Al $\leq$ 1,15).

L'art antérieur n'a reconnu, comme adsorbant pour la séparation des xylènes, de faujasites échangées au baryum qu'avec des rapports atomiques Si/Al entre 1,2 et 2,6. En la matière, l'homme du métier ne semble pas disposer de critère simple et suffisamment fiable pour prévoir leur comportement. Aussi la quête d'adsorbants toujours améliorés procède-t-elle plus ou moins de recherches aléatoires, comme par exemple le résultat qu'expose et revendique US 3.878.127 avec un échange baryum proprement dit de zéolite X préalablement traitée avec une solution de soude. La demanderesse propose des adsorbants offrant des sélectivités paraxylène/métaxylène ou paraxylène/orthoxylène d'au moins 2,0 et avantageusement d'au moins 2,5 mesurées selon le test décrit dans les exemples. L'invention atteint ce résultat, avec quelques avantages qui apparaîtront dans la description.

EXPOSE DE L'INVENTION

La présente invention a pour objet des adsorbants zéolitiques agglomérés avec un liant zéolitisable, qui est une argile de la famille du kaolin, comprenant au moins 70 % et de préférence au moins 80 % de faujasite de rapport atomique Si/Al tel que $1 \leq Si/Al \leq 1,15$, dont les sites échangeables sont occupés au moins à 70% par des ions baryum et éventuellement jusqu'à 30 % par du potassium

(le complément éventuel étant généralement assuré par des ions alcalins ou alcalino-terreux autres que le baryum et le potassium) et au plus 30%, de préférence au plus de 20% de liant zéolitisable, qui est une argile de la famille du kaolin, les faujasites préférées étant celles dont le taux global d'échange en baryum seul ou en baryum+potassium est supérieur ou égal à 90 %.

Un procédé de préparation des adsorbants zéolitiques agglomérés selon l'invention consiste à d'abord agglomérer de la poudre de zéolite de rapport Si/Al tel que $1 \leq Si/Al \leq 1,15$ avec un liant zéolitisable, qui est une argile de la famille du kaolin.

Par agglomération, on entend l'obtention de particules solides à partir d'un mélange de zéolite(s) et de liant(s) au moyen de toutes techniques connues de l'homme de l'art, telles que extrusion, granulation, compactage, atomisation. La teneur pratique en liant de l'aggloméré ne dépasse généralement pas 30 %, et de préférence 20 %, de la masse totale de l'adsorbant. On améliore substantiellement l'efficacité de ces adsorbants en retenant comme liant d'agglomération une argile de la famille du kaolin, en pratique la kaolinite ou l'halloysite, et en soumettant les granulés à zéolitisation.

La zéolitisation du liant est pratiquée par immersion de l'aggloméré dans une liqueur alcaline, soudé ou mélange de soude et de potasse dont la concentration est de préférence au moins 0,5 M, après que les grains aient été calcinés, cette première calcination ayant pour premier résultat de durcir le grain, mais aussi d'activer l'argile en la transformant en métakaolin. La zéolitisation se fait de préférence à chaud, un travail à plus haute température améliorant la cinétique du processus et réduisant les durées d'immersion. On obtient ainsi aisément des zéolitisations d'au moins 50% du liant, c'est-à-dire que l'adsorbant résultant est généralement constitué d'au moins 85 % et de préférence d'au moins 90 % de zéolite de type faujasite active et d'au plus 15 %, de préférence d'au plus 10 %, de matière inactive pour l'adsorption.

L'échange au baryum est pratiqué de façon tout à fait conventionnelle, de préférence par échanges successifs de façon à atteindre un taux d'échange visé minimum d'au moins 70 % et de préférence d'au moins 90%.

L'échange au potassium peut être pratiqué avant ou après l'échange au baryum mais il est également possible d'agglomérer de la poudre de faujasite LSX contenant déjà des ions potassium.

L'activation est la dernière étape de l'obtention des adsorbants de l'invention. Elle a pour but de fixer la teneur en eau, plus simplement la perte au feu de l'adsorbant dans des limites optimales. On procède de la façon la plus pratique par activation thermique qu'on exécute préférentiellement entre 180 et 250°C.

L'invention consiste également en un perfectionnement de procédé de récupération de paraxylène à partir de coupes d'isomères $C_8$ aromatiques consistant à utiliser comme agent d'adsorption un adsorbant zéolitique à base de faujasite de rapport Si/Al tel que $1 \leq Si/Al \leq 1,15$, dont les sites échangeables sont occupés au moins à 70% par des ions baryum (le complément étant assuré par des ions alcalins ou alcalino-terreux autres que le baryum) agglomérée avec un liant zéolitisable, qui est une argile de la famille du kaolin. Les adsorbants zéolitiques agglomérés selon l'invention conviennent lorsqu'ils sont mis en œuvre dans des procédés en phase liquide ou en phase gazeuse.

On peut ainsi séparer le produit désiré par chromatographie liquide d'adsorption préparative (en batch), avantageusement en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :

| | |
|---|---|
| nombre de lits | 6 à 30 |
| nombre de zones | au moins 4 |
| température | 100 à 250°C, de préférence 150 à 190 °C |
| pression | 0,2 à 3 MPa |
| rapport des débits désorbant sur charge | 1 à 2,5 |
| | (par exemple 1,4 à 1,8 pour une unité d'adsorption seule (stand alone) et 1,1 à 1,4 pour une unité d'adsorption combinée à une unité de cristallisation) |

(suite)

| taux de recyclage | 3,5 à 12, de préférence 4 à 6 |
| --- | --- |

On pourra se référer aux brevets US 2.985.589, US 5.284.992 et US 5.629.467.

Les conditions opératoires d'une unité industrielle d'adsorption à co-courant simulé sont en général les mêmes que celles fonctionnant à contre-courant simulé à l'exception du taux de recyclage qui est en général compris entre 0,8 et 7. On pourra se référer aux brevets US 4.402.832 et US 4.498.991.

Le solvant de désorption peut être un désorbant dont le point d'ébullition est inférieur à celui de la charge, tel que le toluène mais aussi un désorbant dont le point d'ébullition est supérieur à celui de la charge, tel que le paradiéthylbenzène (PDEB)

On préfère ici les faujasites de rapport Si/Al sensiblement égal à 1, que l'on prépare selon le mode d'obtention décrit dans le brevet européen EP 486.384 ou le brevet US 5.173.462. La sélectivité des adsorbants selon l'invention pour l'adsorption du p-xylène contenu dans des coupes aromatiques en $C_8$ est optimale lorsque leur perte au feu mesurée à 900 °C est comprise en général entre 4,0 et 7,7 %, et de préférence entre 5,2 et 7,7 %. De l'eau et un peu de dioxyde de carbone rentrent dans la perte au feu.

Les exemples qui suivent, non limitatifs, feront mieux comprendre l'invention.

EXEMPLES

Ces exemples font appel à la mesure ou l'appréciation de certaines grandeurs caractéristiques des adsorbants de l'invention.

Pour apprécier la sélectivité qu'offre l'adsorbant d'un procédé de séparation du paraxylène, on lui applique un test qui permet la mesure de son pouvoir séparateur entre le paraxylène (PX) et ses isomères $C_8$ aromatiques (MX, OX), mais aussi entre paraxylène et éthylbenzène (EB), ce qui est important parce que certaines coupes peuvent être riches en éthylbenzène et ne pas l'être en autres isomères $C_8$, et également entre le paraxylène et le désorbant, parce qu'il est tout aussi important de disposer d'une sélectivité faible PX / désorbant, condition pour que la désorption soit efficace.

Le test consiste à immerger un adsorbant (17 grammes) préalablement activé thermiquement et refroidi à l'abri de l'air, dans 80 g d'un mélange d'aromatiques dissous dans du 2,2,4=triméthylpentane.

La composition exacte du mélange est la suivante :

| | |
| --- | --- |
| PX | 2 % |
| MX | 2 % |
| OX | 2 % |
| EB | 2 % |
| désorbant (toluène ou p-diéthylbenzène) | 2 % |
| 2,2,4-triméthylpentane | le reste |

On procède à l'autoclave à 150°C, pendant 4 heures, durée suffisante pour assurer l'équilibre d'adsorption. Une partie du liquide est alors prélevée, condensée à -30°C et analysée par chromatographie en phase gazeuse. Il est alors possible de remonter aux concentrations dans la phase adsorbée et dans la phase non adsorbée et d'exprimer la quantité de paraxylène adsorbée et les sélectivités en paraxylène par rapport aux autres aromatiques et au désorbant. Le 2,2,4-triméthylpentane ne perturbe pas ces résultats, étant très peu adsorbé.

Pour les exemples 1 à 9, le désorbant mis en oeuvre est le toluène et le paradiéthylbenzène pour l'exemple 10.

EXEMPLE 1 : préparation d'un adsorbant témoin

On agglomère une zéolite NaX industrielle, de rapport Si/Al = 1,25 et de rapport Na/Al = 1, en mélangeant intimement 850 grammes de poudre de zéolite X, (exprimés en équivalent calciné), 150 grammes de kaolinite des Charentes (exprimés en équivalent calciné) et 6 grammes de carboxyméthylcellulose, (adjuvant de rétention destiné à retenir l'eau lors de l'opération d'extrusion) avec la quantité d'eau adéquate pour l'extrusion. L'extrudé est séché, concassé de manière à récupérer des grains dont le diamètre équivalent est égal à 0,7 mm, puis calciné à 550°C sous courant d'azote pendant 2 heures. Sa capacité d'adsorption de toluène, déterminée à 25°C et sous une pression partielle de 0,5, est de 20,2 % ; on l'interprète en volume microporeux de 20,2/0,86 =0,235 $cm^3/g$ (dans le calcul du volume poreux, on considère que la densité de la phase liquide est identique à la densité du toluène adsorbé, c'est-à-dire 0,86). Ce granulé est échangé au moyen d'une solution de chlorure de baryum 0,5 M à 95°C en 4 étapes. A chaque étape, le rapport volume de solution sur masse de solide est de 20 ml/g et l'échange est poursuivi pendant 4 heures à chaque fois. Entre chaque échange, le solide est lavé plusieurs fois de manière à le débarrasser des excédents de sel. Il est ensuite activé à une température de 250°C pendant 2 heures sous courant d'azote. Sa capacité d'adsorption de toluène est de 14,8 %, assimilée à un volume microporeux de 0,17 $cm^3$/g. On mesure également la perte au feu, grandeur importante car elle donne une estimation de l'eau résiduelle présente sur l'adsorbant : on relève ici une perte au feu de 4,5 %.

L'application du test de sélectivité conduit aux résultats suivants :

| Isomères | Sélectivité |
|---|---|
| PX/OX | 2,25 |
| PX/MX | 2,12 |
| PX/EB | 1,77 |
| PX/Tol | 1,52 |

La quantité de paraxylène adsorbé est égale à 0,054 $cm^3$/g.

Le taux effectif de zéolite contenue dans cet adsorbant est proche de 85 %.

EXEMPLE 2 : préparation d'un adsorbant selon l'invention

On agglomère 950 grammes (équivalent calciné) d'une zéolite X de rapport Si/Al = 1,01 obtenue selon le processus décrit dans le brevet européen EP 0486.384 ou le brevet US 5.173.462, avec 170 grammes (équivalent calciné) de kaolinite des Charentes, 6 grammes de carboxyméthylcellulose et la quantité d'eau adéquate pour pouvoir extruder correctement la pâte obtenue. Les extrudés sont ensuite séchés puis calcinés à une température de 600°C pendant 2 heures sous courant d'azote sec. On procède alors à un concassage de manière à ramener le diamètre équivalent des particules à 0,7 mm.

Les concassés ainsi obtenus sont soumis au traitement d'échange baryum déjà décrit dans l'exemple 1 et activés thermiquement à une température de 220°C. Le produit ainsi obtenu a une perte au feu de 5 % et une capacité d'adsorption de toluène de 13 % (volume microporeux 0,15 $cm^3$/g).

L'adsorbant répond au test de sélectivité avec les valeurs suivantes :

| Isomères | Sélectivité |
|---|---|
| PX/OX | 2,60 |
| PX/MX | 2,55 |
| PX/EB | 2,80 |
| PX/Tol | 2,00 |

La quantité de paraxylène adsorbé pendant le test est de 0,057 $cm^3$/g, soit la même que celle mesurée sur l'adsorbant de l'exemple 1 malgré la différence de volume microporeux. On note aussi une meilleure sélectivité vis-à-vis de l'éthylbenzène, ce qui peut être intéressant lorsqu'il faut traiter des charges riches en cet isomère. Le chiffre de la sélectivité du paraxylène vis à vis du toluène est tout à fait favorable à une désorption du paraxylène moyennant une consommation raisonnable en désorbant.

EXEMPLE 3 : préparation d'un adsorbant selon l'invention

Comme précédemment, on agglomère 950 grammes d'une zéolite X de rapport Si/Al = 1,01 avec 170 grammes de kaolinite des Charentes, 6 grammes de carboxyméthylcellulose et la quantité d'eau adéquate. On extrude. Les extrudés sont séchés, calcinés à une température de 600°C pendant 2 heures sous courant d'azote sec, puis concassés de manière à ramener leur diamètre équivalent à 0,7 mm.

On immerge maintenant 10 grammes de ces agglomérés dans 17 ml d'une solution de soude à 220 g/l pendant 3 heures à 95°C. On lave successivement quatre fois à l'eau.

Pour estimer l'efficacité de la zéolitisation, une petite partie du produit est portée à 550°C sous courant d'azote sec et l'on détermine une capacité d'adsorption de toluène de 21,6 %. La teneur globale en zéolite active est estimée à 95 %, c'est à dire supérieure à sa teneur initiale dans l'adsorbant aggloméré.

Le solide est ensuite échangé au baryum dans les mêmes conditions que celles exposées dans l'exemple 1. On mesure après activation sous azote sec à 220°C pendant 2 heures une capacité d'adsorption de toluène de 15 % (volume microporeux : 0,175 cm$^3$/g) et une perte au feu de 5,2 %.

L'adsorbant ainsi préparé est évalué selon le test de sélectivité.

On obtient :

| Isomères | Sélectivité |
|----------|-------------|
| PX/OX | 2,64 |
| PX/MX | 2,60 |
| PX/EB | 2,75 |
| PX/Tol | 1,94 |

La quantité de paraxylène adsorbée pendant le test est égale à 0,066 cm$^3$/g. Les sélectivités vis à vis des différents isomères se comparent bien à celle de l'adsorbant de l'exemple 2, ce qui traduit que l'élément actif des deux produit est une zéolite LSX. Le gain substantiel de paraxylène adsorbé est la conséquence de la richesse en LSX du fait de la zéolitisation du liant

EXEMPLES 4 A 7 : préparation et test d'adsorbants selon l'invention ayant subi diverses activations finales

On répète la préparation d'échantillon comme dans l'exemple 3, à la seule différence que l'on a fait varier la température d'activation entre 180° et 300°C : 180°C pour l'exemple 4, 200°C, pour l'exemple 5, 220°C (repris à l'exemple 3), 250°C pour l'exemple 6, 300°C pour l'exemple 7.

Les caractéristiques comparées de ces produits sont portées au tableau ci-dessous.

| Exemples | 4 | 5 | 3 | 6 | 7 |
|----------|------|------|------|------|------|
| Température d'activation (°C) | 180 | 200 | 220 | 250 | 300 |
| Micro-porosité. | 0,132 | 0,17 | 0,175 | 0,178 | 0,185 |
| Paraxylène adsorbé | 0,0506 | 0,065 | 0,066 | 0,062 | 0,051 |
| PX/OX | 2,91 | 4,21 | 2,64 | 2,17 | 1,45 |
| PX/MX | 3,06 | 3,11 | 2,60 | 2,01 | 1,55 |
| PX/EB | 2,00 | 2,37 | 2,75 | 2,76 | 2,34 |
| PX/Tol | 2,28 | 1,55 | 1,94 | 1,90 | 1,19 |
| Perte au feu (%) | 7,7 | 6,6 | 5,2 | 4,4 | 2,4 |

L'objectif préféré de sélectivité PX/OX et PX/MX d'au moins 2,5 est satisfait pour ces zéolites lorsque la perte au feu (en gros, la teneur en eau) mesurée après l'étape d'activation du produit échangé est de 5,2 à 7,7 %, ce qu'on atteint pratiquement par activation thermique à une température située entre 180 et 220°C.

L'objectif préféré de sélectivité PX/EB d'au moins 2,5 est satisfait pour ces zéolites lorsque la perte au feu (en gros, la teneur en eau) est de 4,4 à 5,2 %, ce qu'on atteint pratiquement par activation thermique à une température située entre 220 et 250°C.

L'activation à 250°C altère quelque peu ces performances, le produit restant néanmoins intéressant pour traiter des coupes plutôt riches en éthylbenzène.

EXEMPLE 8

On prépare des échantillons d'agglomérés comme indiqué à l'exemple 3 que l'on échange au potassium puis selon le mode opératoire ci-dessous :

Sur un aggloméré dont le liant a été zéolitisé, on opère un échange poussé au potassium au moyen d'une solution de KCl 1 M à 25 °C en 4 étapes successives. A chaque étape, le rapport volume de solution sur masse de solide est de 20 ml/g et l'échange est poursuivi pendant 4 heures à chaque fois. Entre chaque échange, le solide est lavé plusieurs fois de manière à le débarrasser des excédents de sel. Le produit obtenu présente un taux d'échange en potassium de 97,5 %. On lui fait alors subir 2 opérations d'échange au baryum identiques à celles décrites à l'exemple 1. Après toutes ces opérations, le solide est enfin activé à une température de 200°C pendant 2 heures sous courant d'azote. Il présente les caractéristiques suivantes :

| | |
|---|---|
| taux d'échange en baryum | 74,3 % |
| taux d'échange en potassium | 24 % |
| capacité d'adsorption en toluène | 15 % |
| volume microporeux | 0,174 cm$^3$/g |
| perte au feu à 900 °C | 6,4 % |

L'adsorbant ainsi préparé répond au test de sélectivité avec les valeurs suivantes :

| Isomères | Sélectivité |
|---|---|
| PX/OX | 3,82 |
| PX/MX | 3,01 |
| PX/EB | 2,42 |
| PX/Tol | 1,72 |

La quantité de paraxylène adsorbé pendant le test est de 0,06 cm$^3$/g.

EXEMPLE 9

On agglomère 950 grammes (équivalent calciné) d'une zéolite X de rapport Si/Al = 1,12 dont la synthèse est inspirée de "Investigation on the crystallization of X-type zeolites" de H. Lechert, Zeolites, 1991, vol. 11 pp. 720-728, avec 170 grammes (équivalent calciné) de kaolinite des Charentes, 6 grammes de carboxyméthylcellulose et la quantité d'eau adéquate pour pouvoir extruder correctement la pâte obtenue. Les extrudés sont ensuite séchés puis calcinés à une température de 600°C pendant 2 heures sous courant d'azote sec. On procède alors à un concassage de manière à ramener le diamètre équivalent des particules à 0,7 mm.

Les concassés ainsi obtenus sont soumis au traitement d'échange baryum décrit dans l'exemple 1 et activés thermiquement à une température de 220°C pendant 2 heures. Le produit ainsi obtenu a une perte au feu de 5,2 % et une capacité d'adsorption de toluène de 13,7 % (volume microporeux 0,159 cm$^3$/g).

L'adsorbant répond au test de sélectivité avec les valeurs suivantes :

| Isomères | Sélectivité |
|---|---|
| PX/OX | 2,52 |
| PX/MX | 2,50 |
| PX/EB | 2,62 |
| PX/Tol | 1,78 |

La quantité de paraxylène adsorbé pendant le test est de 0,059 cm$^3$/g,

EXEMPLE 10

L'adsorbant préparé à l'exemple 3 est soumis à un test de sélectivité identique en utilisant le paradiéthylbenzène comme désorbant et on trouve les valeurs suivantes:

| Isomères | Sélectivité |
|---|---|
| PX/OX | 2,65 |
| PX/MX | 2,58 |

(suite)

| Isomères | Sélectivité |
|----------|-------------|
| PX/EB | 2,70 |
| PX/PDEB | 1,12 |

## Revendications

1. Adsorbants zéolitiques agglomérés avec un liant comprenant au moins 70 % et de préférence au moins 80 % de faujasite de rapport atomique Si/Al tel que $1 \leq Si/Al \leq 1,15$ dont les sites échangeables sont occupés au moins à 70% par des ions baryum et éventuellement jusqu'à 30 % par du potassium (le complément éventuel étant généralement assuré par des ions alcalins ou alcalino-terreux autres que le baryum et le potassium) et au plus 30%, de préférence au plus 20% de liant, le liant étant un liant zéolitisable, qui est une argile de la famille du kaolin.

2. Adsorbants selon la revendication 1 dont le taux global d'échange en baryum seul est supérieur ou égal à 90 %.

3. Adsorbants selon la revendication 1 dont le taux global d'échange en baryum+potassium est supérieur ou égal à 90 %.

4. Adsorbants selon la revendication 1 à 3 dont la perte au feu mesurée à 900°C est comprise entre 4,0 et 7,7 %, et de préférence entre 5,2 et 7,7 %.

5. Procédé d'obtention des adsorbants tels que définis dans l'une quelconque des revendications 1 à 4 comprenant les étapes suivantes :

   a/agglomération de poudre de zéolite avec un liant zéolitisable, qui est une argile de la famille du kaolin,
   b/calcination de l'aggloméré,
   c/zéolitisation éventuelle du liant par immersion de l'aggloméré dans une liqueur alcaline, soude ou mélange de soude et de potasse,
   d/échange au baryum et éventuellement au potassium e/activation.

6. Procédé d'obtention d'adsorbants selon la revendication 5, **caractérisé en ce que** l'activation à l'étape e/est une activation thermique exécutée à une température de 180 à 250°C.

7. Procédé de récupération de paraxylène à partir de coupes d'isomères C8 aromatiques en phase liquide, par adsorption du paraxylène au moyen d'un adsorbant selon l'une quelconque des revendications 1 à 4 en présence d'un désorbant.

8. Procédé de récupération de paraxylène selon la revendication 7 de type lit mobile simulé.

9. Procédé de récupération de paraxylène selon la revendication 8 de type contre-courant simulé.

10. Procédé de récupération de paraxylène selon la revendication 8 de type co-courant simulé.

11. Procédé de récupération de paraxylène à partir de coupes d'isomères C8 aromatiques en phase gazeuse, par adsorption du paraxylène au moyen d'un adsorbant selon l'une quelconque des revendications 1 à 4 en présence d'un désorbant.

12. Procédé de récupération de paraxylène selon l'une quelconque des revendications 7 à 11 dans lequel le désorbant est le toluène ou le paradiéthylbenzène.

## Patentansprüche

1. Zeolithische, mit einem Bindemittel agglomerierte Adsorptionsmittel, enthaltend wenigstens 70 %, bevorzugt wenigstens 80 %, Faujasit mit einem Atomverhaltnis Si/Al von $1 \leq Si/Al \leq 1.15$, dessen austauschbare Stellen zuwenigstens 70 % durch Barium-Ionen und gegebenenfalls bis zu 30 % durch Kalium besetzt sind (wobei gegebenenfalls das Komplement im Allgemeinen durch alkalische oder erdalkalische Ionen, bei denen es sich nicht um Barium und Kalium handelt, sicher gestellt wird), und hochstens 30 %, bevorzugt hochstens 20 %, Bindemittel, mit einem zeolithisierbaren Bindemittel, der ist einem Ton aus der Kaolin-Familie.

2. Adsorptionsmittel gemaß Anspruch 1, deren Gesamtaustauschgrad an Barium allein 90 % oder mehr betragt.

3. Adsorptionsmittel gemaß Anspruch 1, deren Gesamtaustauschgrad an Barium + Kalium 90 % oder mehr betragt.

4. Adsorptionsmittel gemaß einem der Anspruche 1 bis 3, deren bei 900°C gemessener Abbrandverlust zwischen 4,0 und 7,7 %, bevorzugt zwischen 5,2 und 7,7 %, liegt.

5. Verfahren zum Erhalt von Adsorptionsmitteln wie in irgendeinem der Anspruche 1 bis 4 definiert, welches folgende Schritte umfasst:

   a/ Agglomeration von Zeolithpulver mit einem zeolithisierbaren Bindemittel, der ist einem Ton aus der Kaolin-Familie,
   b/ Kalzinieren des Agglomerats,
   c/ gegebenenfalls Zeolithisierung des Bindemittels durch Eintauchen des Agglomerats in eine alkalische Flussigkeit, Natriumcarbonat oder eine Mischung aus Natriumcarbonat und Kaliumcarbonat,
   d/ Austausch mit Barium und, gegebenenfalls, Kalium,
   e/ Aktivierung.

6. Verfahren zum Erhalt von Adsorptionsmitteln gemaß Anspruch 5, **dadurch gekennzeichnet, dass** die Aktivierung in Schritt e/ eine thermische Aktivierung ist, die bei einer Temperatur von 180 bis 250 ° c durchgefuhrt wird.

7. Verfahren zur Wiedergewinnung von Paraxylol ausgehend von Fraktionen von $C_8$-aromatischen Isomeren in Flussigphase durch Adsorption von Paraxylol mittels eines Adsorptionsmittels gemaß irgendeinem der Anspruche 1 bis 4 in Gegenwart eines Desorptionsmittels.

8. Verfahren zur Wiedergewinnung von Paraxylol gemaß Anspruch 7 vom simulierten FlieBbett-Typ.

9. Verfahren zur Wiedergewinnung von Paraxylol gemaß Anspruch 8 vom simulierten Gegenstrom-Typ.

10. Verfahren zur Wiedergewinnung von Paraxylol gemaß Anspruch 8 vom simulierten Mitstrom-Typ.

11. Verfahren zur Wiedergewinnung von Paraxylol ausgehend von Schnitten von $C_8$-aromatischen Isomeren in Gasphase durch Adsorption von Paraxylol mittels eines Adsorptionsmittels gemaß irgendeinem der Anspruche 1 bis 4 in Gegenwart eines Desorptionsmittels.

12. Verfahren zur Wiedergewinnung von Paraxylol gemaß irgendeinem der Anspruche 7 bis 11, wobei das Desorptionsmittel Toluol oder Paradiethylbenzol ist.

**Claims**

1. Agglomerated zeolite adsorbents with a binder comprising at least 70%, and preferably at least 80% of faujasite with an Si/Al atomic ratio such that $1 \leq Si/Al \leq 1.15$, in which exchangeable sites of the zeolite adsorbents are at least 70% occupied by barium ions, and optionally up to 30% occupied by potassium (any remainder generally being made up of alkali metal ions or alkaline-earth metal ions other than barium and potassium) and at most 30%, preferably at most 20% of the binder, the binder being a zeolitizable binder, which is a clay of the kaolin family.

2. Adsorbents according to claim 1, wherein an overall level of exchange for barium alone is greater than or equal to 90%.

3. Adsorbents according to claim 1, wherein an overall level of exchange for barium and potassium is greater than or equal to 90%.

4. Adsorbents according to claim 1 to 3, wherein the loss on ignition measured at 900°C is between 4.0 and 7.7%, and preferably 5.2 and 7.7%.

5. Process for obtaining the adsorbents defined in anyone of claims 1 to 4, comprising the following steps:

a) agglomerating zeolite powder with a zeolitizable binder, which is a clay of the kaolin family,
b) calcining the agglomerate,
c) optional zeolitization of the binder by immersing the agglomerate in an alkaline liquor, sodium hydroxide or a mixture of sodium hydroxide and potassium hydroxide,
d) exchanging the exchangeable sites with barium, and optionally with potassium, and
e) activating the adsorbents.

6. Process for obtaining adsorbents according to claim 5, wherein the activation in step e) is a thermal activation performed at a temperature of 180 to 250°C.

7. Process for the recovery of para-xylene from aromatic C8 isomer fractions in a liquid phase, comprising adsorbing the para-xylene using the adsorbents according to anyone of claims 1 to 4 in the presence of a desorbent.

8. Process for the recovery of para-xylene according to claim 7, wherein the adsorbing occurs in a simulated fluid-bed.

9. Process for the recovery of para-xylene according to claim 8, wherein the adsorbing occurs in a simulated counter-current.

10. Process for the recovery of para-xylene according to claim 8, wherein the adsorbing occurs in a simulated co-current.

11. Process for the recovery of para-xylene from aromatic C8 isomer fractions in a gaseous phase, comprising adsorbing the para-xylene using the adsorbents according to anyone of claims 1 to 4 in the presence of a desorbent.

12. Process for the recovery of para-xylene according to anyone of claims 7 to 11, wherein the desorbent is toluene or para-diethylbenzene.

## RÉFÉRENCES CITÉES DANS LA DESCRIPTION

**Documents brevets cités dans la description**

- US 3558730 A
- US 3558732 A
- US 3626020 A
- US 3663638 A
- US 2985589 A
- US 2882244 A
- US 3130007 A
- US 3878127 A

- US 5284992 A
- US 5629467 A
- US 4402832 A
- US 4498991 A
- EP 486384 A
- US 5173462 A
- EP 0486384 A

**Littérature non-brevet citée dans la description**

- **H. LECHERT.** Investigation on the crystallization of X-type zeolites. *Zeolites,* 1991, vol. 11, 720-728